# EUROPEAN PATENT APPLICATION

(11) **EP 2 209 003 A1**
(43) Date of publication of application: **21.07.2010**
(21) Application number: 09150706.1
(22) Date of filing: 16.01.2009
(51) Int. Cl.: G01N 33/68

(54) **Means and methods for differentiating between fibrosis and cirrhosis**

(71) Applicant: F. Hoffmann-La Roche AG, 4070 Basel (CH); Roche Diagnostics GmbH, 68305 Mannheim (DE)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention is concerned with a method for differentiating between liver fibrosis and liver cirrhosis in a subject based on the determination of Growth Differentiation Factor 15 (GDF-15) and/or Endoglin in a sample of a subject and comparing the, thus, determined amount with a reference amount. Further envisaged by the present invention are kit and a device adapted to carry out the method of the present invention.

## Description

The present invention is concerned with a method for differentiating between liver fibrosis and liver cirrhosis in a subject based on the determination of Growth Differentiation Factor 15 (GDF-15) and/or Endoglin in a sample of said subject and comparing the, thus, determined amount with a reference amount. Further envisaged by the present invention are kit and a device adapted to carry out the method of the present invention.

The liver is the largest internal organ in the body and is involved in glycogen storage, degradation of red blood cells, plasma protein synthesis, and removal of drugs, alcohol and other harmful substances from the blood. Life without the liver is not possible; a subject can only survive a few hours without liver function.

The most abundant liver cells are hepatocytes which comprise approximately 70 % of the liver's mass. Blood supply for the liver comes from the hepatic artery, which supplies oxygen-rich blood. The liver also gets blood from the portal vein which drains blood from the digestive system and its associated glands.

Liver fibrosis (also known as hepatic fibrosis) occurs in most types of chronic liver diseases. It is an exuberant wound healing in which fibrotic tissue is abnormally accumulated in the liver as a result of major liver tissue injury (for a review, see e.g. Bataller and Brenner, 2006, Journal of Clinical Investigation, 115(2) 209-217).

Liver fibrosis is thought to be initiated by activation of the hepatic perivascular stellate cells. These stellate cells (and also adjacent cells) proliferate, and become myofibroblasts (and, thus, contractile cells). Myofibroblasts produce excessive amounts of abnormal matrix (consisting mainly of collagen, but also of other glycoproteins and glycans) and matricellular proteins. In the following Kupffer cells, injured hepatocytes, platelets, and leukocytes aggregate.

The developing myofibroblasts are stimulated by endothelin and increase portal vein resistance and the density of the abnormal matrix. Fibrous tracts join branches of afferent portal veins and efferent hepatic veins which bypass the hepatocytes and limit blood supply of the hepatocytes. Thus, fibrosis may result in hepatocyte ischemia and portal hypertension.

Liver fibrosis can be caused by various drugs and disorders. For example, liver fibrosis is caused by liver diseases such as viral hepatitis B, chronic viral hepatitis C, alcoholic liver disease non-alcoholic fatty liver disease, autoimmune liver disease (such as autoimmune hepatitis and primary biliary cirrhosis), inherited metabolic disorders (such as hematochromatosis), Wilson's disease, alfa 1-antitrypsin deficiency, and drug or toxin-induced liver disease.

The gold standard for diagnosing liver fibrosis is liver biopsy for which a small sample of liver tissue is removed from the patient and analyzed under the microscope. Histologic examination of liver tissue allows for staging liver fibrosis and for assessing the underlying disorder (e.g. viral hepatitis).

Liver fibrosis can be classified into various stages depending on the degree of fibrosis. The stage of liver fibrosis can be assessed by using well known scales such as Metavir (stages I-IV) and Ishak score (stages I-V), see e.g., Thein et al., 2008, Hepatology Vol. 48, No. 2:418-431; or Morra et al., 2007, Expert Rev. Mol. Diagn. 7(5) 481-490.

Specific staining of extracellular matrix proteins can be used to quantify the degree of fibrosis, using computer-guided morphometric analysis (see Bataller and Brenner, loc. cit.). However, histologic examination of liver tissues is cost-intensive and requires trained personnel. Moreover, a liver biopsy must be obtained by an invasive procedure which may cause pain or even major complications. Also, histologic examination of liver tissue is known to be error-prone (e.g. due to sampling errors or due to wrong interpretation of the biopsy). Biochemical markers are under study but are not ready for routine clinical use.

Adams (Clinical Chemistry, 2005;51:1867-1873) proposes a model based on the so called hepascore (also called fibroscore) for staging liver fibrosis. The model provides clinically useful information regarding different fibrosis stages among hepatitis C patients. However, determining the hepascore includes the determination of four serum markers (α₂-Macroglobulin, Bilirubin, GGT, and Hyaluronic acid) plus age and sex.

Liver cirrhosis might evolve from liver fibrosis. It is a progressive, fibrosing and nodular condition disrupting the normal architecture of the liver. In liver cirrhosis liver tissue is replaced with non-living scar tissue as well as with regenerative nodules resulting in decreased liver function. Cirrhosis can be caused by the same disorders and drugs as liver fibrosis. Alcohol abuse is the most common cause for liver cirrhosis. Approximately 60 to 70 percent of newly diagnosed cases of cirrhosis are thought to be a consequence of chronic alcoholism (alcohol liver disease). Regardless of the cause of cirrhosis, the pathogenic features consist of the development of fibrosis to the point that there is architectural distortion with the formation of regenerative nodules surrounded by dense fibrotic tissue.

Liver cirrhosis can be diagnosed by various diagnostic methods. As for liver fibrosis, the gold standard is liver biopsy for which a small sample of liver tissue is removed from the patient and analyzed under the microscope. Cirrhosis can also be diagnosed by various imaging techniques such as ultrasound, computerized tomography (CT) scan and magnetic resonance imaging (MRI).

Moreover, various blood tests for diagnosing liver cirrhosis were introduced during the last two decades (for a review, see Heidelbauch and Bruderly, 2006, American Family Physican, 74(5):756-762). These blood tests are based on the determination of the amount of certain polypeptides which are present or absent in liver cirrhosis. For example, cirrhosis results in reduced levels of blood albumin and in reduced levels of blood clotting factors as a consequence of the loss of the liver's ability to generate these proteins. Moreover, increased levels of the serum enzymes aspartate transaminase, alanin transaminase, alkaline phosphatase and gamma glutamyl-transferase may indicate liver cirrhosis. Another diagnostic assay is the bilirubin test. Bilirubin is a degradation product of the breakdown of blood cells. Impaired liver tissue can not sufficiently process bilirubin, leading to high blood levels of this pigment.

Liver fibrosis may be asymptomatic. Most of the related morbidity and mortality occurs after the development to liver cirrhosis. In the majority of patients, development to liver cirrhosis occurs after approximately 15 to 20 year. Liver cirrhosis, however, may also be asymptomatic for years (and 30% patients never develop symptoms).

Treatment of liver cirrhosis and liver fibrosis may differ. Therefore, it is necessary to identify those patients which suffer from liver cirrhosis and those which suffer from liver fibrosis. However, differentiation between liver cirrhosis and liver fibrosis is mostly conducted by histologic examination which is known to be error-prone, time-consuming and cost-intensive (see above).

Therefore, there is a clear long-standing need for means and methods allowing a differential diagnosis between liver fibrosis and liver cirrhosis in a subject. The said means and methods shall allow a reliable efficient diagnosis and shall avoid the drawbacks of the current techniques.

Thus, the technical problem underlying the present invention must be seen as the provision of means and methods for complying with the aforementioned needs.

The technical problem is solved by the embodiments characterized in the claims and herein below.

Accordingly, the present invention relates a method for differentiating between liver fibrosis and liver cirrhosis in a subject, comprising the steps of
a) determining the amount of GDF-15 (Growth Differentiation Factor 15) and/or Endoglin in a sample of said subject,
b) comparing the amount of GDF-15 and/or Endoglin with a reference amount, and
c) differentiating between liver fibrosis and liver cirrhosis based on the results of step b).

The method of the present invention, preferably, is an in vitro method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) and/or (c) may in total or in part be assisted by automation, e.g., by a suitable robotic and sensory equipment for the determination in step (a) or a computer-implemented comparison in step (b).

The term "differentiating" as used herein means to distinguish between liver fibrosis and liver cirrhosis in a subject. The term as used herein, preferably, includes differentially diagnosing liver fibrosis and liver cirrhosis in a subject. Thus, the method of the present invention allows for assessing whether a subject suffers from liver cirrhosis or liver fibrosis. Diagnosing as used herein refers to assessing the probability according to which a subject suffers from the diseases referred to in this specification. As will be understood by those skilled in the art, such an assessment is usually not intended to be correct for 100% of the subjects to be differentially diagnosed. The term, however, requires that a statistically significant portion of subjects can be correctly diagnosed. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "subject" as used herein relates to animals, preferably mammals, and, more preferably, humans. However, it is envisaged by the present invention that the subject shall, preferably, suffer liver disease. More preferably, said subject shall suffer from liver fibrosis or liver cirrhosis.

A "liver disease" in the context of the method of the present invention, preferably, may be any disease of liver that causes liver fibrosis and liver cirrhosis. Such diseases are well known in the art (see, e.g. Bataller, loc. cit.). Preferably, the liver disease is selected from the group of chronic viral hepatitis B, chronic viral hepatitis C, alcoholic liver disease, non-alcoholic fatty liver disease, autoimmune liver disease (such as autoimmune hepatitis and primary biliary cirrhosis), inherited metabolic liver disorders (such as hematochromatosis), Wilson's disease, alfa 1-antitrypsin deficiency, and drug or toxin-induced liver disease. The most preferred liver diseases are chronic viral hepatitis B, chronic viral hepatitis C, alcoholic liver disease, and non-alcoholic fatty liver disease.

Fibrosis is pathological condition in which fibrous connective tissue invades an organ, usually as a consequence of inflammation or other injury. The term "liver fibrosis" is known by the skilled person. Liver fibrosis (herein also referred to as hepatic fibrosis) is a chronic liver disease in which fibrotic tissue is abnormally accumulated in the liver as a result of major liver tissue injury. The accumulation of extracelluar matrix (ECM) proteins (mostly collagen) during the progression distorts the hepatic architecture (for a review, see e.g. Bataller and Brenner, 2006, Journal of Clinical Investigation, 115(2) 209-217 which hereby is incorporated by reference with respect to its entire disclosure content). It is to be understood that the term "liver fibrosis", as used herein, preferably, does not encompass "liver cirrhosis".

Liver fibrosis can be diagnosed by well known methods. Moreover, liver fibrosis can be classified into various stages depending on the degree of fibrosis. The stage of liver fibrosis can be assessed, e.g. by using well known scales such as Metavir (stages I-IV) and Ishak score (stages I-V), see Thein et al., 2008, Hepatology Vol. 48, No. 2:418-431; or Morra et al., 2007, Expert Rev. Mol. Diagn. 7(5) 481-490. Specific staining of extracellular matrix proteins can be used to quantify the degree of fibrosis, using computer-guided morphometric analysis (see Bataller and Brenner, loc. cit.).

The term "liver cirrhosis" is understood by the skilled person. As used herein, the term, preferably, refers to a liver disease in which the normal microcirculation, the gross vascular anatomy, and the hepatic architecture have been variably destroyed and altered with fibrous septa surrounding regenerated or regenerating parenchymal nodules. Liver cirrhosis occurs in an advanced stage of liver fibrosis. Thus, liver cirrhosis is a progressive, fibrosing and nodular condition disrupting the normal architecture of the liver. In liver cirrhosis liver tissue is replaced with non-living scar tissue as well as with regenerative nodules resulting in decreased liver function. Thus, cirrhosis occurs when the liver is inflamed and scar tissue and regenerative nodules form. Thus, liver cirrhosis occurs when pathogenic fibrosis develops to the point that there is architectural distortion in the liver with the formation of regenerative nodules surrounded by dense fibrotic tissue (see Harrison's Principles of Internal Medicine, 17th edition, editor A. Fauci, Mcgraw-Hill Professional, particularly chapter 302).

As set forth above, liver cirrhosis may evolved from liver fibrosis. In the context of the method of the present invention liver cirrhosis, preferably, differs from liver fibrosis by the presence of regenerated nodules and by the distortion of the normal hepatic architecture.

Liver cirrhosis can be graded by the Child-Pugh classification system (also called Child-Pugh-Score or Child-Turcotte-Pugh). Said system is based on assessing five variables of liver cirrhosis, i.e. ascites, the plasma concentrations of bilirrubin and albumin, the prothrombin time, and the degree of encephalopathy. It allows for allocating patients with liver disease into three groups (Child A, B and C) depending on the severity of cirrhosis, see also Pugh et al., Transection of the oesophagus for bleeding oesophageal varices. Br J Surg 1973; 60:646-649). Accordingly, a subject suffering from liver cirrhosis, preferably suffers from liver disease graded Child A, B or C by the Child-Pugh classification system.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein. More preferably, cell-, tissue- or organ samples are obtained from liver cells, liver tissue or the liver. The most preferred sample is the context of the present invention is a blood, blood serum, or a blood plasma sample.

The term "Growth-Differentiation Factor-15" or "GDF-15" relates to a polypeptide being a member of the transforming growth factor (TGF)-β cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated genre-1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as an approx. 28 kDa dimeric protein (Bauskin 2000, Embo J 19:2212-2220). Amino acid sequences for GDF-15 are disclosed in WO99/06445, WO00/70051, WO2005/113585, Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The term "Endoglin" as used herein refers to a polypeptide having a molecular weight of 180 kDa non-reduced, 95 kDa after reduction and 66 kDa in its reduced and N-deglycosylated form. The polypeptide is capable of forming dimers and binds to TGF-β and TGF-β receptors (see below). Endoglin may be phosphorylated. Preferably, Endoglin refers to human Endoglin. More preferably, human Endoglin has an amino acid sequence as shown in GenBank accession number AAC63386.1, GI: 3201489. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific Endoglin (how to determine the degree of identity is described herein above). Variants may be allelic variants, splice variants or any other species specific homologs, paralogs, or orthologs. Moreover, the variants referred to herein include fragments of the specific Endoglin or the aforementioned types of variants as long as these fragments have the essential immunological and biological properties as referred to above. Such fragments may be, e.g., degradation products of Endoglin. Further included are variants which differ due to posttranslational modifications such as phosphorylation or myristylation.

Determining the amount of the polypeptides referred to in this specification (particularly, Endoglin and GDF-15) relates to measuring the amount or concentration, preferably semi-quantitatively or quantitatively. Measuring can be done directly or indirectly. Direct measuring relates to measuring the amount or concentration of the polypeptide based on a signal which is obtained from the polypeptide itself and the intensity of which directly correlates with the number of molecules of the polypeptide present in the sample. Such a signal - sometimes referred to herein as intensity signal -may be obtained, e.g., by measuring an intensity value of a specific physical or chemical property of the polypeptide. Indirect measuring includes measuring of a signal obtained from a secondary component (i.e. a component not being the polypeptide itself) or a biological read out system, e.g., measurable cellular responses, ligands, labels, or enzymatic reaction products.

In accordance with the present invention, determining the amount of a polypeptide can be achieved by all known means for determining the amount of a polypeptide in a sample.

Said means comprise immunoassay devices and methods which may utilize labeled molecules in various sandwich, competition, or other assay formats. Said assays will develop a signal which is indicative for the presence or absence of the polypeptide. Moreover, the signal strength can, preferably, be correlated directly or indirectly (e.g. reverse- proportional) to the amount of polypeptide present in a sample. Further suitable methods comprise measuring a physical or chemical property specific for the polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a polypeptide comprises the steps of (a) contacting a cell capable of eliciting a cellular response the intensity of which is indicative of the amount of the polypeptide with the said polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the polypeptide.

Also preferably, determining the amount of a polypeptide comprises the step of measuring a specific intensity signal obtainable from the polypeptide in the sample. As described above, such a signal may be the signal intensity observed at an mass to charge (m/z) variable specific for the polypeptide observed in mass spectra or a NMR spectrum specific for the polypeptide.

Determining the amount of a polypeptide may, preferably, comprises the steps of (a) contacting the polypeptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the polypeptide described herein. Preferred ligands include antibodies, nucleic acids, peptides or polypeptides such as receptors or binding partners for the polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the polypeptide. Specific binding according to the present invention means that the ligand or agent should not bind substantially to ("cross-react" with) another peptide, polypeptide or substance present in the sample to be analyzed. Preferably, the specifically bound polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative. Suitable methods are described in the following.
First, binding of a ligand may be measured directly, e.g. by NMR or surface plasmon resonance.
Second, if the ligand also serves as a substrate of an enzymatic activity of the polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/polypeptide" complex or the ligand which was bound by the polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.
Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a polypeptide may be, also preferably, determined as follows: (a) contacting a solid support comprising a ligand for the polypeptide as specified above with a sample comprising the polypeptide and (b) measuring the amount of polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide, the relative amount or concentration of the said polypeptide as well as any value or parameter which correlates thereto or can be derived therefrom. Such values or parameters comprise intensity signal values from all specific physical or chemical properties obtained from the said polypeptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

Comparing as used herein encompasses comparing the amount of the polypeptides referred to herein which are comprised by the sample to be analyzed with an amount of the said polypeptides in a suitable reference sample as specified below in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison by means of an expert system. Accordingly, a differential diagnosis for the liver cirrhosis and liver fibrosis referred to herein may be automatically provided in a suitable output format.

The term "reference amount" as used herein refers to an amount which allows for differentiating between liver cirrhosis and liver fibrosis as referred to above. How to determine suitable reference amounts is well known in the art. The reference may either be derived from a subject known to suffer from liver cirrhosis or from a subject known to suffer from liver fibrosis. It is to be understood that if a reference sample from a subject is used which suffers from liver cirrhosis, an amount of the polypeptides in a sample of a test subject being essentially identical to the amounts determined in the said reference sample (i.e. the reference amounts) shall be indicative for liver cirrhosis. Likewise, if a reference sample from a subject is used which suffers from liver fibrosis, an amount of the polypeptides in a sample of a test subject being essentially identical to the amounts determined in the said reference sample (i.e. the reference amounts) shall be indicative for liver fibrosis. The reference amount applicable for an individual subject may vary depending on various physiological parameters such as age, gender, or subpopulation and on the technique used for assessing the amount. Thus, a suitable reference amount may be determined by the method of the present invention from a reference sample to be analyzed together, i.e. simultaneously or subsequently, with the test sample.

Moreover, a threshold amount can be preferably used as a reference amount. An amount of the polypeptides which is above the threshold amount or below the threshold amount will be indicative for either liver cirrhosis or liver fibrosis. Preferably, an amount of the polypeptides which is above the threshold amount will be indicative for liver cirrhosis. Preferably, an amount of the polypeptides which is below the threshold amount will be indicative for liver fibrosis.

A preferred reference amount for GDF-15 in the context of the present invention defining a threshold amount for GDF-15 as referred to in accordance with the present invention is, preferably, within a range of 1500 to 2500 pg/ml, and, more preferably, within a range of 1500 to 2000 pg/ml. Particularly preferred reference amounts are 1500 pg/ml, 2000 pg/ml, or 2500 pg/ml.

Preferably, an amount of GDF-15 in a sample a subject larger than the reference amount for GDF-15 (defining a threshold amount for GDF-15) is indicative for liver cirrhosis.

Preferably, an amount of GDF-15 in a sample a subject lower than the reference amount for GDF-15 (defining a threshold amount for GDF-15) is indicative for liver fibrosis.

A preferred reference amount for Endoglin in the context of the present invention defining a threshold amount for Endoglin as referred to in accordance with the present invention is, preferably, within a range of 5.5 to 8 ng/ml, and, more preferably, within a range of 6 to 7 ng/ml. Particularly preferred reference amounts are 6 ng/ml, 7 ng/ml, or 8 ng/ml.

Preferably, an amount of Endoglin in a sample a subject larger than the reference amount for Endoglin (defining a threshold amount for Endoglin) is indicative for liver cirrhosis.

Preferably, an amount of Endoglin in a sample a subject lower than the reference amount for Endoglin (defining a threshold amount for Endoglin) is indicative for liver fibrosis.

The following applies, if both Endoglin and GDF-15 are determined.

Preferably, an amount of GDF-15 and Endoglin in a sample a subject larger than the reference amount for GDF-15 and Endoglin, respectively, is indicative for liver cirrhosis.

Preferably, an amount of GDF-15 and Endoglin in a sample a subject lower than the reference amount for GDF-15 and Endoglin, respectively, is indicative for liver fibrosis.

Advantageously, it has been found in the studies underlying the present invention that determining the amount of GDF-15 and/or Endoglin in a sample of a subject, and comparing the, thus, determined amount(s) with a reference amount (reference amount) allows for reliably differentiating between liver fibrosis and liver cirrhosis. Specifially, the amounts of GDF-15 and Endoglin were determined in serum samples in 64 patients. Of these patients, 31 patients suffered from liver cirrhosis and 34 from liver fibrosis. Subjects with liver fibrosis were staged according to the Ishak score (Ishak et al., Histological grading and staging of chronic hepatitis. J Hepatol 1995; 22:696-699). Subjects with liver cirrhosis were staged by determining the Child-Pugh-Score (Child A: 11 subjects, Child B: 11 subjects, Child C: 9 subjects). It was shown that the amounts of Endoglin and GDF-15 were significantly increased in subjects with liver cirrhosis compared with subjects with liver fibrosis (see Examples).

It is to be understood that either Endoglin or GDF-15 alone, or both Endoglin and GDF-15 can be determined in order to differentiate between liver cirrhosis and liver fibrosis.

It is to be understood that based on whether a subject suffers from liver cirrhosis or liver cirrhosis an appropriate therapy can be chosen. Thanks to the present invention, subjects can be more readily and reliably diagnosed and subsequently correctly treated according to the result of the said differential diagnosis.

The explanations given herein above apply mutatis mutandis to the following.

Moreover, the present invention relates to a method for diagnosing liver cirrhosis in a subject with liver disease comprising the steps
a) determining the amount of GDF-15 (Growth Differentiation Factor 15) and/or Endoglin in a sample of said subject,
b) comparing the amount of GDF-15 and/or Endoglin with a reference amount, and
c) diagnosing liver cirrhosis based on the results of step b).

Preferred reference amounts for GDF-15 and Endoglin are disclosed elsewhere herein.

Preferably, an amount of GDF-15 in a sample a subject larger than the reference amount for GDF-15 (defining a threshold amount for GDF-15 as referred to elsewhere herein) indicates that said subject suffers from liver cirrhosis.

Preferably, an amount of GDF-15 in a sample a subject lower than the reference amount for GDF-15 (defining a threshold amount for GDF-15) indicates that said subject does not suffer from liver cirrhosis.

Preferably, an amount of Endoglin in a sample a subject larger than the reference amount for Endoglin (defining a threshold amount for Endoglin) indicates that said subject suffers from liver cirrhosis.

Preferably, an amount of Endoglin in a sample a subject lower than the reference amount for GDF-15 (defining a threshold amount for Endoglin) indicates that said subject does not suffer from liver cirrhosis.

The following applies, if both Endoglin and GDF-15 are determined.

Preferably, an amount of GDF-15 and Endoglin in a sample a subject larger than the reference amount for GDF-15 and Endoglin, respectively, indicates that said subject suffers from liver cirrhosis.

Preferably, an amount of GDF-15 and Endoglin in a sample a subject lower than the reference amount for GDF-15 and Endoglin, respectively, indicates that said subject does not suffer from liver cirrhosis.

Moreover, the present invention relates to a device for differentiating between liver fibrosis and liver cirrhosis comprising
a) means for determining the amount(s) of GDF-15 and/or Endoglin in a sample of a subject, and
b) means for comparing the amount(s) determined by the means of a) with a reference amount (reference amounts), whereby it is differentiated between liver fibrosis and liver cirrhosis.

Moreover, the present invention relates to a device for diagnosing liver cirrhosis in a subject with liver disease, comprising
a) means for determining the amount(s) of GDF-15 and/or Endoglin in a sample of a subject, and
b) means for comparing the amount(s) determined by the means of a) with a reference amount (reference amounts), whereby liver cirrhosis is diagnosed.

The term "device" as used herein relates to a system of means comprising at least the aforementioned means operatively linked to each other as to allow the prediction. Preferred means for determining the amount of the said polypeptides and means for carrying out the comparison are disclosed above in connection with the method of the invention. How to link the means in an operating manner will depend on the type of means included into the device. For example, where means for automatically determining the amount of the peptides are applied, the data obtained by said automatically operating means can be processed by, e.g., a computer program in order to diagnose or distinguish between the diseases referred to herein. Preferably, the means are comprised by a single device in such a case. Said device may accordingly include an analyzing unit for the measurement of the amount of the peptides in a sample and a computer unit for processing the resulting data for the differential diagnosis. Alternatively, where means such as test stripes are used for determining the amount of the polypeptides, the means for diagnosing may comprise control stripes or tables allocating the determined amount to an amount known to be accompanied with pulmonary embolism or pulmonary hypertension as the cause of right heart failure. The test stripes are, preferably, coupled to a ligand which specifically binds to the polypeptides as defined elsewhere in this specification. The strip or device, preferably, comprises means for detection of the binding of said peptides to the said ligand. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisage are expert system devices wherein the output comprises processed diagnostic raw data the interpretation of which does not require a specialized clinician. Further preferred devices comprise the analyzing units/devices (e.g., biosensors, arrays, solid supports coupled to ligands specifically recognizing the polypeptides, Plasmon surface resonace devices, NMR spectrometers, mass- spectrometers etc.) or evaluation units/devices referred to above in accordance with the method of the invention.

Moreover, the present invention relates to a kit for differentiating between liver fibrosis and liver cirrhosis, wherein said kit comprises instructions for carrying out the said differentiation, and
a) means for determining the amount(s) of GDF-15 and/or Endoglin in a sample of a subject, and
b) means for comparing the amount(s) determined by the means of a) with a reference amount (reference amounts), whereby it is differentiated between liver fibrosis and liver cirrhosis.

Moreover, the present invention relates to a kit for diagnosing liver cirrhosis in a subject with liver disease, wherein said kit comprises instructions for carrying out the said diagnosis, and
a) means for determining the amount(s) of GDF-15 and/or Endoglin in a sample of a subject, and
b) means for comparing the amount(s) determined by the means of a) with a reference amount (reference amounts), whereby liver cirrhosis is diagnosed.

The term "kit" as used herein refers to a collection of the aforementioned means, preferably, provided in separately or within a single container. The container, also preferably, comprises instructions for carrying out the method of the present invention. The invention, thus, relates to a kit comprising a means or an agent for measuring a polypeptide referred to herein. Examples for such means or agents as well as methods for their use have been given in this specification. The kit, preferably, contains the aforementioned means or agents in a ready-to-use manner. Preferably, the kit may additionally comprise instructions, e.g., a user's manual for interpreting the results of any determination(s) with respect to the diagnoses provided by the methods of the present invention. Particularly, such manual may include information for allocating the amounts of the determined polypeptides to the kind of diagnosis. Details are to be found elsewhere in this specification. Additionally, such user's manual may provide instructions about correctly using the components of the kit for determining the amount(s) of the respective biomarker. A user's manual may be provided in paper or electronic form, e.g., stored on CD or CD ROM. The present invention also relates to the use of said kit in any of the methods according to the present invention.

Moreover, the present invention relates to the use of GDF-15 and/or Endoglin in a sample of a subject for differentiating between liver fibrosis and liver cirrhosis.

Moreover, the present invention relates to the use of GDF-15 and/or Endoglin in a sample of a subject for diagnosing liver cirrhosis.

All references cited in this specification are herewith incorporated by reference with respect to their entire disclosure content and the disclosure content specifically mentioned in this specification.

### The Figures show:

**Fig 1****:** GDF-15 levels in subjects with liver fibrosis or liver cirrhosis (Box plot diagram, see also Example 1)
**Fig 2****:** Endoglin levels in subjects with liver fibrosis or liver cirrhosis (Box plot diagram, see also Example 1)

The following Examples merely illustrate the invention. It shall, whatsoever, not be construed as to limit the scope of the invention.

### Example 1

The amounts of GDF-15 and Endoglin were determined in serum samples of 64 patients suffering liver fibrosis and liver cirrhosis.

The patient collective is the same as decribed by Raedle-Hurst et al. (Europoean Journal of Gastroenterology & Hepatology 2008, Vol 20 No 9).

Mean age was 48.1 +/- 6.5 years (range 31-65). Twenty six patients were female and 38 were male.

A hepatic biopsy was performed in all patients except those with manifest signs of liver cirrhosis (see Raedle-Hurst et al., loc. cit). Liver biopsy specimens were assessed by an experienced pathologist who was unaware of the clinical and biochemical data of the patients. Fibrosis of the liver was staged according to the Ishak score (Ishak et al., Histological grading and staging of chronic hepatitis. J Hepatol 1995; 22:696-699). Mild fibrosis refers to stage F1-2, moderate fibrosis to F3-4, and severe fibrosis/cirrhosis to F5-6. Severe fibrosis/cirrhosis was classified according to the Child-Turcotte-Pugh criteria (Pugh et al., Transection of the oesophagus for bleeding oesophageal varices. Br J Surg 1973; 60:646-649).

Staging of liver disease indicated mild fibrosis (F1-2) in 25 of 64 (39.1%) patients, moderate fibrosis (F3-4) in 8 of 64 (12.5%) patients, and severe fibrosis/cirrhosis (F5-6) in 31 of 64 (48.4%) patients. In the subgroup of patients with severe fibrosis/cirrhosis, the degree of liver dysfunction was classified as Child-Turcotte-Pugh class A in 11 patients, B in 11 patients, and C in 9 patients.

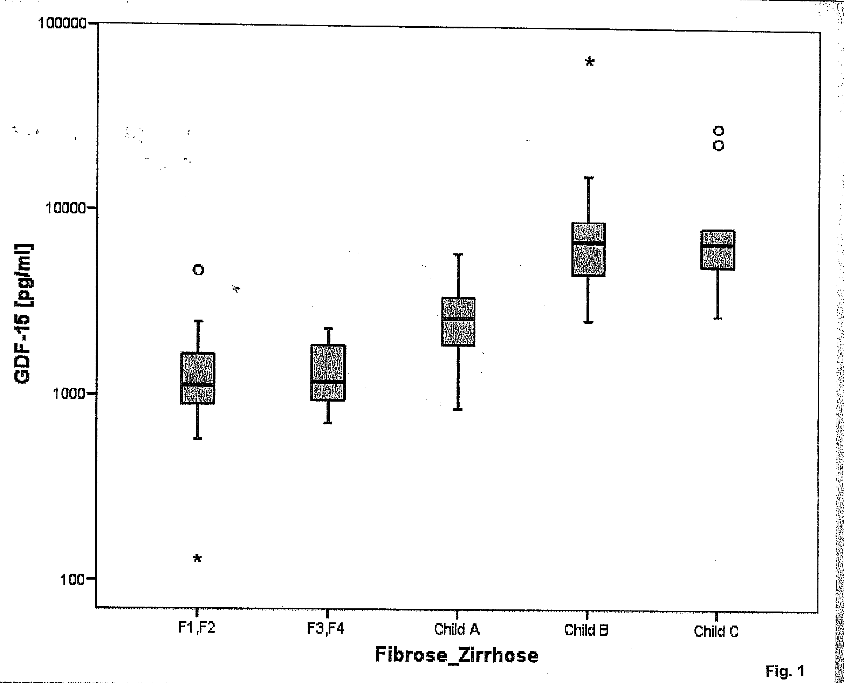

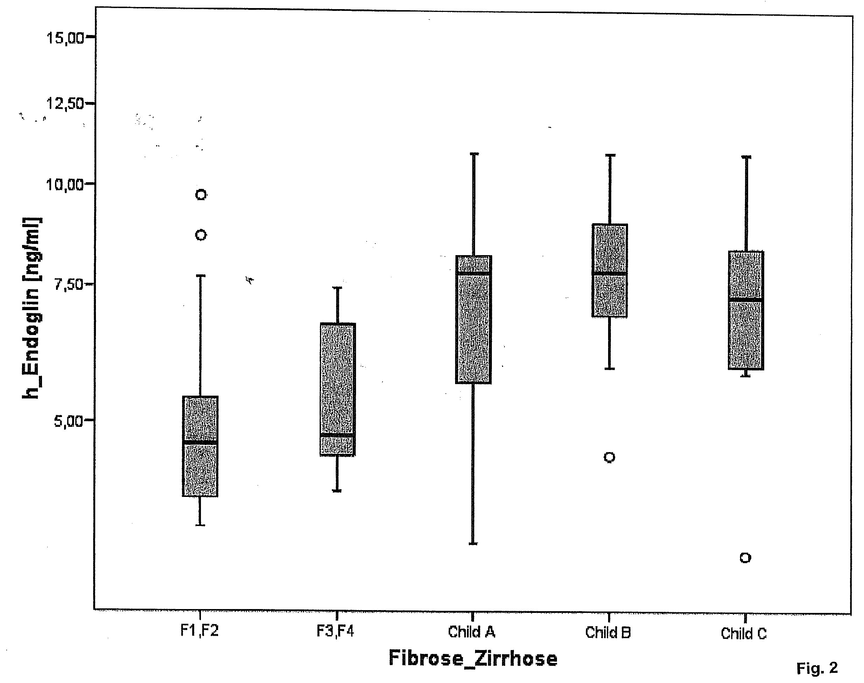

The results are shown in Fig. 1 and Fig. 2.

As it can be seen from the results, the amounts of Endoglin and GDF-15 are larger in samples from patients with cirrhosis than in samples from patients with fibrosis. Accordingly, the determination of these markers allows for differentiating between liver cirrhosis and liver fibrosis.

### Example 2

A 59 years old winemaker presents at his primary care physician for routine examination. In the past, there have been no indications for a pathological condition except for elevated elevated levels of Serum glutamic oxaloacetic transaminase (SGOT) and Gamma-glytamyl transpeptidase (gamma GT) which, however, were thought to result from overweight (body mass index: 27). In a further examination, GDF-15 (2530 pg/ml) and Endoglin (6 ng/ml) are determined in a serum sample obtained from the patient. A subsequent ultrasonic examination indicates the presense of liver disease. A liver biopsy is carried out and liver cirrhosis is diagnosed (due to the presence of small inactive nodules).

### Example 3

Endoglin and GDF-15 are determined in a serum sample from a 49 years old male patient with chronic hepatitis C (genotype 1): Endoglin 4.2 ng/ml, GDF-15 1320 pg/ml. The patient is treated with interferon alpha and Ribavirin. Although the treatment reduces the amount of HCV RNA (viral load), elimination of HCV is not achieved after 6 months of treatment. Then, treatment with interferon is stopped. After two more years, Endoglin and GDF-15 are determined again in a serum sample (Endoglin 6.1 ng/ml, GDF-15 1810 pg/ml). Examination of liver biopsies obtained at treatment initiation and two years after treatment stopped shows the progression from fibrosis to cirrhosis.

### Example 4

GDF-15 was determined by an electrochemoluminescence immunoassay the Elecsys^{™} instrument (Roche Diagnostics GmbH, Mannheim, Germany). The assay works according to the electrochemoluminescence sandwich immunoassay principle. In a first step, the biotin-labelled IgG (1-21) capture antibody, the ruthenium-labelled F(ab')₂ (39-50) signal antibody and 20 microliters of sample are incubated at 37 °C for 9 minutes. Afterwards, streptavidin-coated magnetic microparticles are added and the mixture is incubated for additional 9 minutes. After the second incubation, the reaction mixture is transferred to the measuring cell of the system where the beats are magnetically captured onto the surface of an electrode. Unbound label is removed by washing the measuring cell with buffer.

In the last step, voltage is applied to the electrode in the presence of a tri-propylamine containing buffer and the resulting electrochemoluminescent signal is recorded by a photomultiplier. All reagents and samples are handled fully automatically by the Elecsys^{™} instrument. Results are determined via a calibration curve which is instrument-specifically generated by 2-point calibration and a master curve provided via the reagent barcode. The test is performed according to the instructions of the manufacturer.

Plasma levels of Endoglin were determined using the commercially available Immunoassays "Quantikine" (Catalog numbers DNDG00) from R & D Systems, USA

## Claims

1. A method for differentiating between liver fibrosis and liver cirrhosis in a subject, comprising the steps of
a) determining the amount of GDF-15 (Growth Differentiation Factor 15) and/or Endoglin in a sample of said subject,
b) comparing the amount of GDF-15 and/or Endoglin with a reference amount, and
c) differentiating between liver fibrosis and liver cirrhosis based on the results of step b).

2. The method of claim 1, wherein said subject is human.

3. The method of claim 1 and 2, wherein said sample is blood, blood serum, or a blood plasma sample.

4. The method of any one of claims 1 to 3, wherein the amount of GDF-15 is determined and wherein an amount of GDF-15 larger than the reference amount is indicative for liver cirrhosis.

5. The method of any one of claims 1 to 3, wherein GDF-15 is determined, and wherein an amount of GDF-15 lower than the reference amount is indicative for liver fibrosis.

6. The method of claim 4 or 5, wherein the reference amount of GDF-15 is 2000 pg/ml.

7. The method of any one of claims 1 to 3, wherein Endoglin is determined and wherein an amount of Endoglin larger than the reference amount is indicative for liver cirrhosis.

8. The method of any one of claims 1 to 3, wherein Endoglin is determined, and wherein an amount of Endoglin lower than the reference amount is indicative for liver fibrosis.

9. The method of claim 7 or 8, wherein the reference amount of Endoglin is 6 ng/ml.

10. The method of any one of claims 1 to 3, wherein the amount of GDF-15 and the amount of Endoglin is determined.

11. A device for differentiating between liver fibrosis and liver cirrhosis comprising
a) means for determining the amount(s) of GDF-15 and/or Endoglin in a sample of a subject, and
b) means for comparing the amount(s) determined by the means of a) with a reference amount (reference amounts), whereby it is differentiated between liver fibrosis and liver cirrhosis.

12. A kit for differentiating between liver fibrosis and liver cirrhosis, wherein said kit comprises instructions for carrying out the said differentiation, and
a) means for determining the amount(s) of GDF-15 and/or Endoglin in a sample of a subject, and
b) means for comparing the amount(s) determined by the means of a) with a reference amount (reference amounts), whereby it is differentiated between liver fibrosis and liver cirrhosis.
